(19) Europäisches Patentamt
European Patent Office
Office européen des brevets

(11) Veröffentlichungsnummer: **0 071 095**
**B1**

(12) **EUROPÄISCHE PATENTSCHRIFT**

(45) Veröffentlichungstag der Patentschrift: **05.12.84**

(51) Int. Cl.³: **C 07 D 249/08**, C 07 D 233/60, A 01 N 43/64, A 01 N 43/50

(21) Anmeldenummer: **82106336.9**

(22) Anmeldetag: **15.07.82**

(54) **Phenylketenacetale und diese enthaltende Fungizide.**

(30) Priorität: **31.07.81 DE 3130215**

(43) Veröffentlichungstag der Anmeldung: **09.02.83 Patentblatt 83/6**

(45) Bekanntmachung des Hinweises auf die Patenterteilung: **05.12.84 Patentblatt 84/49**

(84) Benannte Vertragsstaaten: **AT BE CH DE FR GB IT LI NL SE**

(56) Entgegenhaltungen:
**EP - A - 0 022 975**
**EP - A - 0 023 651**
**GB - A - 1 318 590**

(73) Patentinhaber: **BASF Aktiengesellschaft, Carl-Bosch-Strasse 38, D-6700 Ludwigshafen (DE)**

(72) Erfinder: **Ruland, Alfred, Dr., Schriesheimer Strasse 11, D-6945 Hirschberg (DE)**
Erfinder: **Reuther, Wolfgang, Dr., Am Pferchelhang 16, D-6900 Heidelberg (DE)**
Erfinder: **Ammermann, Eberhard, Dr., Sachsenstrasse 3, D-6700 Ludwigshafen (DE)**
Erfinder: **Pommer, Ernst-Heinrich, Dr., Berliner Platz 7, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 071 095 B1

**Beschreibung**

Die vorliegende Erfindung betrifft neue Phenylketen-O,N- und -S,N-acetale und ihre Verwendung als Fungizide sowie diese enthaltende Fungizide.

Es ist bekannt, 1-[2'-(2,4-Dichlorphenyl)-2'-(2-propenyloxy)ethyl]-1H-imidazol (GB-PS Nr. 1318590) als Fungizid zu verwenden. Seine Wirkung ist jedoch unbefriedigend.

Es ist ferner bekannt, Triazolylalkenderivate oder Imidazolylenolether als Fungizide zu verwenden (EP-A Nrn. 22975 und 23651). Diese Verbindungen haben jedoch eine andere Struktur als die neuen Verbindungen. Die neuen Verbindungen werden daher durch die bekannten Verbindungen nicht nahegelegt.

Es wurde nun gefunden, dass Phenylketen-O,N- und -S,N-acetale der allgemeinen Formel 1:

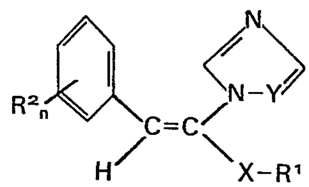
(1)

in welcher

$R^1$ Alkyl mit 1 bis 6 C-Atomen, Cycloalkyl mit 3 bis 8 C-Atomen, Naphthyl oder Phenyl bedeutet, wobei der Phenylrest durch Alkyl oder Alkoxy mit jeweils 1 bis 5 C-Atomen, Fluor, Chlor, Brom, Jod, Trifluormethyl oder durch Phenoxy substituiert sein kann,

$R^2$ Wasserstoff, Alkyl oder Alkoxy mit jeweils 1 bis 5 C-Atomen, Fluor, Chlor, Brom, Jod oder Trifluormethyl bedeutet,

n 1 oder 2 bedeutet,

Y für N und X für O oder S steht,

und deren Salze und Metallkomplexe eine überraschend gute fungizide Wirkung haben.

$R^1$ bedeutet beispielsweise Methyl, Ethyl, 4-Bromphenyl, 3-Chlorphenyl, 2,4-Dichlorphenyl, 4-Chlorphenyl.

$R^2$ bedeutet beispielsweise Methyl.

Salze sind beispielsweise die Salze mit Salzsäure, Bromwasserstoffsäure, Schwefelsäure, Salpetersäure, Phosphorsäure, Dodecylbenzolsulfonsäure.

Metallkomplexe sind beispielsweise die Metallkomplexe mit Kupfer(II)sulfat, Kupfer(II)nitrat, Zink(II)chlorid, Eisen(III)chlorid, Mangan(II)-chlorid, Nickel(II)bromid.

Es wurde ferner gefunden, dass die Verbindungen der allgemeinen Formel 1 sehr leicht und in guten Ausbeuten durch Zersetzung aus den Sulfonaten der Formel 2: →

→

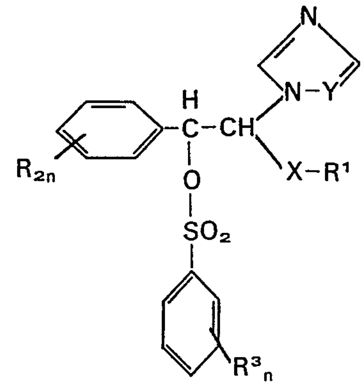
(2)

hergestellt werden können, in der $R^1$, $R^2$, n, X und Y die oben angegebene Bedeutung haben, und $R^3$, Wasserstoff, Halogen oder Alkyl mit 1 bis 5 C-Atomen bedeutet.

Die Umsetzung wird in Anwesenheit eines Lösungsmittels, z.B. Dimethylformamid, Dimethylsulfoxid, Hexamethylphosphorsäuretriamid, Tetrahydrofuran oder Dioxan, und von basischen Katalysatoren, wie Alkalihydroxiden, -sulfiden, -carbonaten, -alkoholaten oder -phenolaten, vorzugsweise Natriumsulfid, Natriumethylenat und Lithium-tert.-butanolat, bei Temperaturen zwischen 10 und 100°C durchgeführt.

Die Salze werden durch Umsetzung eines Acetals mit einer Säure, die Metallkomplexe durch Umsetzung eines Acetals mit einem Metallsalz z.B. Kupfersulfat hergestellt.

Die für die Umsetzung benötigten Sulfonate der Formel 2 lassen sich aus den entsprechenden Metallalkoholaten 3, die nach bekannten Verfahren hergestellt werden können und in der Literatur beschrieben sind, durch Umsetzung mit den Sulfonsäurechloriden 4 gewinnen,

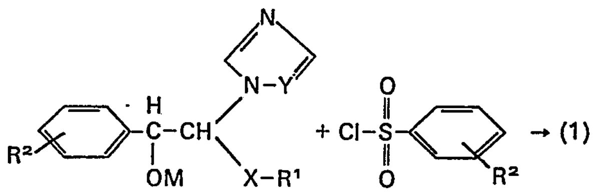
(3) (4)

wobei $R^1$, $R^2$, X und Y die oben genannten Bedeutungen haben und M ein Alkalimetall z.B. Na, K bedeutet.

Die Verbindungen der Formel 1 können als E- oder Z-Isomere vorliegen, je nach Anordnung der Gruppen an den C-Atomen der Doppelbindung. Geht man von diastereoisomerenreinen Sulfonaten 2 aus, lassen sich selektiv die E- bzw. Z-Isomere erhalten. Sowohl die reinen Isomere als auch ihre Gemische werden von der Erfindung umfasst.

*Beispiel 1:*

*1-[1,2,4-Triazolyl-(1)]-1-(2,4-dichlorphenoxy)-2-phenylethylen (Verbindung 3)*

A) 35 g (0,1 mol) 1-[1,2,4-Triazolyl-(1)]-1-(2,4-dichlorphenoxy)-2-phenylethan-2-ol werden in 500 ml absolutem Tetrahydrofuran (THF) mit 2,4 g (0,1 mol) Natriumhydrid bei 30 bis 40°C unter Stickstoff umgesetzt. Nach 3 h kühlt man auf Raumtemperatur (20°C), versetzt mit 19,1 g (0,1 mol) p-Toluolsulfochlorid, lässt noch 1 h rühren, hydrolysiert anschliessend mit Wasser, extrahiert mehrfach mit Essigester, trocknet die organische Phase über Natriumsulfat und destilliert das Lösungsmittel im Vakuum ab. Das so erhaltene Rohtosylat wird aus Essigester/Cyclohexan umkristallisiert. Ausbeute: 30,2 g (60% d.Th.), Schmp.: 126-128°C.

*Analyse für* $C_{23}H_{19}Cl_2N_3O_4S$ (SO4,4):

| | | | | | |
|---|---|---|---|---|---|
| Ber.: | C 54,8 | H 3,8 | Cl 14,1 | N 8,3 | S 6,4% |
| Gef.: | C 54,9 | H 3,9 | Cl 14,1 | N 8,5 | S 6,4% |

B) 25,2 g (0,05 mol) des Tosylats werden in 200 ml Dimethylsulfoxid gelöst und bei Raumtemperatur mit 20 g Natriumsulfid versetzt. Sobald die zunächst auftretende Grünfärbung verschwunden ist, wird mit 500 ml Wasser versetzt, zweimal mit 500 ml Ether extrahiert, über Natriumsulfat getrocknet und im Vakuum eingeengt. Man erhält 13,3 g (80% d.Th.) der gewünschten Verbindung 3 als farbloses Öl ($^1$H-NMR-Daten, s. Tabelle).

In entsprechender Weise werden die folgenden Verbindungen hergestellt.

| Verbindung Nr. | $R^1$ | $R^2$ | X | Y | $^1$H-NMR-Daten (δ in ppm, LM: $CDCl_3$, 60 MHz) |
|---|---|---|---|---|---|
| 1 | 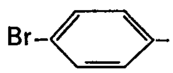 | H | O | N | δ = 6,2 (s, 1H); 6,7-7,7 (m, 9H), 7,9 (s, 1H); 8,2 (s, 1H) |
| 2 | 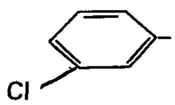 | H | O | N | δ = 6,3 (s, 1H); 6,65-7,5 (m, 9H), 8,0 (s, 1H); 8,05 (s, 1H) |
| 3 | 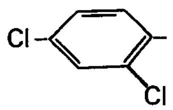 | H | O | N | δ = 6,7-7,6 (m, 9H); 7,9 (s, 1H); 8,2 (s, 1H) |
| 4 | 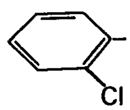 | H | O | N | δ = 6,0 (s, 1H); 6,6-7,6 (m, 9H), 7,9 (s, 1H); 8,2 (s, 1H) |
| 5 | 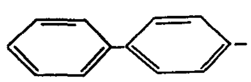 | H | O | N | δ = 6,2 (s, 1H); 6,8-7,7 (m, 14H), 7,9 (s, 1H); 8,2 (s, 1H) |
| 6 | 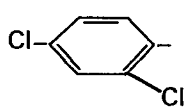 | 4-F | O | N | δ = 6,5 (s, 1H); 6,8-7,8 (m, 8H); 8,45 (s, 2H) |
| 8 | $-CH_3$ | 2,4-$Cl_2$ | O | N | δ = 3,9 (s, 3H); 5,8 (s, 1H), 6,8-7,5 m (m, 3H); 7,9 (s, 2H) |
| 9 | 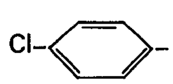 | H | S | N | δ = 7-7,8 (m, 10H) 8,3 (s, 2H) |

Die neuen Verbindungen zeichnen sich durch eine hervorragende Wirksamkeit gegen ein breites Spektrum von pflanzenpathogenen Pilzen, insbesondere aus der Klasse der Ascomyzeten und Basidiomyzeten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zukkerrohr, Obst und Zierpflanzen im Gartenbau, sowie in Gemüsen wie Gurken, Bohnen oder Kürbisgewächsen.

Die neuen Verbindungen sind insbesondere geeignet zur Bekämpfung folgender Pflanzenkrankheiten

*Erysiphe graminis* (echter Mehltau) in Getreide,
*Erysiphe cichoriacearum* (echter Mehltau) an Kürbisgewächsen,
*Podosphaera leucotricha* an Äpfeln,
*Uncinula necator* an Reben,
*Erysiphe polygoni* an Bohnen,
*Sphaerotheca pannosa* an Rosen,
*Puccinia*-Arten an Getreide,
*Rhizoctonia solani* an Baumwolle sowie
*Helminthosporium*-Arten an Getreide,
*Ustilago*-Arten an Getreide und Zuckerrohr,
*Rhynchosporium secale* an Getreide und ganz besonders
*Venturia inaequalis* (Apfelschorf).

Die Verbindungen werden angewendet, indem man die Pflanzen mit den Wirkstoffen besprüht oder bestäubt oder die Samen der Pflanzen mit den Wirkstoffen behandelt. Die Anwendung erfolgt

vor oder nach der Infektion der Pflanzen oder Samen durch die Pilze.

Die erfindungsgemässen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen/Fettalkohol/Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,01 und 3 kg Wirkstoff oder mehr je Hektar. Die neuen Verbindungen können auch in Materialschutz u.a. zur Bekämpfung holzzerstörender Pilze wie *Coniophora puteanea* und *Polystictus versicolor* eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, dass man das Holz mit diesen Mitteln behandelt, beispielsweise tränkt oder anstreicht.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Giessen.

Beispiele für solche Zubereitungen sind:

I. Man vermischt 90 Gew.-Teile der Verbindung des Beispiels 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung des Beispiels 3 werden in einer Mischung gelöst, die aus 80 Gew.-Teilen Xylol, 20 Gew.-Teilen des Anlagerungsproduktes von 8 bis 10 mol Ethylenoxid an 1 mol Ölsäure-N-monoethanolamid, 5 Gew.-Teilen Calciumsalz der Dodecylbenzolsulfonsäure und 5 Gew.-Teilen des Anlagerungsproduktes von 40 mol Ethylenoxid an 1 mol Ricinusöl besteht. Durch Ausgiessen und feines Verteilen der Lösung in Wasser erhält man eine wässerige Dispersion.

III. 20 Gew.-Teile der Verbindung des Beispiels 4 werden in einer Mischung gelöst, die aus 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl besteht. Durch Eingiessen und feines Verteilen der Lösung in Wasser erhält man eine wässerige Dispersion.

IV. 20 Gew.-Teile der Verbindung des Beispiels 4 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl besteht. Durch Eingiessen und feines Verteilen der Lösung in Wasser erhält man eine wässerige Dispersion.

VI. 3 Gew.-Teile der Verbindung des Beispiels 3 werden mit 97 Gew.-Teilen feinteiligem Kaolin innig vermischt. Man erhält auf diese Weise ein Stäubemittel, das 3 Gew.-% des Wirkstoffs enthält.

VII. 30 Gew.-Teile der Verbindung des Beispiels 4 werden mit einer Mischung aus 92 Gew.-Teilen pulverförmigen Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung des Beispiels 3 werden mit 10 Teilen Natriumsalz eines Phenolsulfonsäure/Harnstoff/Formaldehyd-Kondensates, 2 Teilen Kieselgel und 48 Teilen Wasser innig vermischt. Man erhält eine stabile wässerige Dispersion.

IX. 20 Teile der Verbindung des Beispiels 4 werden mit 2 Teilen Calciumsalz der Dodecylbenzolsulfonsäure, 8 Teilen Fettalkoholpolyglykolether, 2 Teilen Natriumsalz eines Phenolsulfonsäure/Harnstoff/Formaldehyd-Kondensates und 68 Teilen eines paraffinischen Mineralöls innig vermischt. Man erhält eine stabile ölige Dispersion.

Die erfindungsgemässen Mittel können in diese Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrösserung des fungiziden Wirkungsspektrums.

Die folgende Liste von Fungiziden, mit denen die erfindungsgemässen Verbindungen kombiniert werden können, soll die Kombinationsmöglichkeiten erläutern, nicht aber einschränken.

Fungizide, die mit den erfindungsgemässen Verbindungen kombiniert werden können, sind beispielsweise

Schwefel,

Dithiocarbamate und deren Derivate, wie

Ferridimethyldithiocarbamat,
Zinkdimethyldithiocarbamat,
Manganethylenbisdithiocarbamat,
Manganzinkethylendiaminbisdithiocarbamat und
Zinkethylenbisdithiocarbamat,
Tetramethylthiuramdisulfide,
Ammoniakkomplex von Zink-(N,N-ethylenbisdithiocarbamat) und N,N'-Polyethylenbis-(thiocarbamoyl)disulfid,
Zink-(N,N'-propylenbisdithiocarbamat),
Ammoniakkomplex von Zink-(N,N'-propylenbisdithiocarbamat) und N,N'-Polypropylenbis-(thiocarbamoyl)disulfid;
Nitroderivate, wie
Dinitro-(1-methylheptyl)phenylcrotonat,
2-sek.-Butyl-4,6-dinitrophenyl-3,3-dimethylacrylat,
2-sek.-Butyl-4,6-dinitrophenylisopropylcarbonat;
heterocyclische Substanzen, wie
N-(1,1,2,2-Tetrachlorethylthio)-tetrahydrophthalimid,
N-Trichlormethylthiotetrahydrophthalimid,
N-Trichlormethylthiophthalimid,
2-Heptadecyl-2-imidazolinacetat,
2,4-Dichlor-6-(o-chloranilino)-s-triazin,
O,O-Diethylphthalimidophosphonothioat,
5-Amino-1-[bis(dimethylamino)phosphinyl]-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazolcarbaminsäuremethylester,
2-Methoxycarbonylaminobenzimidazol,
2-Rhodanmethylthiobenzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-[Furyl-(2)]-benzimidazol,
N,N'-1,4-Piperazindiylbis-[1-(2,2,2-trichlorethyliden)bisformamid],
2-[Thiazolyl-(4)]-benzimidazol,
5-Butyl-2-dimethylamino-4-hydroxy-6-methylpyrimidin,
Bis-(p-chlorphenyl)-3-pyridinmethanol,
1,2-Bis-(3-ethoxycarbonyl-2-thioureido)benzol,
1,2-Bis-(3-methoxycarbonyl-2-thioureido)-benzol;
und weiter Substanzen wie
Dodecylguanidinacetat,
3-[3-(3,5-dimethyl-2-oxycyclohexyl)-2-hydroxyethyl]glutaramid,
Hexachlorbenzol,
N-Dichlorfluormethylthio-N',N'-dimethyl-N-phenylschwefelsäurediamid,
2,5-Dimethylfuran-3-carbonsäureanilid,
2,5-Dimethylfuran-3-carbonsäurecyclohexylamid,
2-Cyan-N-(ethylaminocarbonyl)-2-(methoxyimino)acetamid,
2-Methylbenzoesäureanilid,
2-Iodbenzoesäureanilid,
1-(3,4-Dichloranilino)-1-formylamino-2,2,2-trichlorethan,
2,6-Dimethyl-N-tridecylmorpholin bzw. dessen Salze,
2,6-Dimethyl-N-cyclododecylmorpholin bzw. dessen Salze.
D,L-Methyl-N-(2,6-dimethylphenyl)-N-furoyl-(2)-alaninat,
D,L-N-(2,6-Dimethylphenyl)-N-(2'-methoxyacetyl)alaninmethylester,
5-Nitroisophthalsäurediisopropylester,
1-(1',2',4'-Triazolyl-1')-1-(4'-chlorphenoxy)-3,3-dimethylbutan-2-on,
1-(1',2',4'-Triazolyl-1')-1-(4'-chlorphenoxy)-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff.

Die folgenden Versuche 1, 2 und 3 zeigen die fungizide Wirkung der neuen Substanzen. Als Vergleichssubstanz diente {1-[2'-(2'',4''-Dichlorphenyl)-2'-(2''-propenyloxy)ethyl]-1H-imidazol} A.

*Versuch 1*

*Wirksamkeit gegen Weizenmehltau*

Blätter von in Töpfen gewachsenen Weizenkeimlingen der Sorte Jubilar werden mit wässerigen Emulsionen aus 80% (Gew.-%) Wirkstoff und 20% Emulgiermittel besprüht und 24 h nach dem Antrocknen des Spritzbelages mit Oidien (Sporen) des Weizenmehltaus (*Erysiphe graminis* var. *tritici*) bestäubt. Die Versuchspflanzen werden anschliessend im Gewächshaus bei Temperaturen zwischen 20 und 22°C und 75 bis 80% relativer Luftfeuchtigkeit aufgestellt. Nach 7 d wird das Ausmass der Mehltauentwicklung ermittelt.

Das Ergebnis des Versuches zeigt, dass beispielsweise der Wirkstoff 3 beispielsweise als 0,025, 0,006 und 0,0015%ige Wirkstoffbrühe eine bessere fungizide Wirkung hat (beispielsweise 100%ige Wirkung) als der bekannte Wirkstoff A (beispielsweise 70%ige Wirkung).

*Versuch 2*

*Wirksamkeit gegen Weizenbraunrost*

Blätter von in Töpfen gewachsenen Weizensämlingen der Sorte Jubilar werden mit Sporen des Braunrostes (*Puccinia recondita*) bestäubt. Danach werden die Töpfe für 24 h bei 20 bis 22°C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschliessend mit wässerigen Spritzbrühen, die 80% Wirkstoff und 20% Emulgiermittel in der Trockensubstanz enthalten, tropfnass gespritzt. Nach dem Antrocknen des Spritzbelages werden die

Versuchspflanzen im Gewächshaus bei Temperaturen zwischen 20 und 22° C und 65 bis 70% relativer Luftfeuchte aufgestellt. Nach 8 d wird das Ausmass der Rostpilzentwicklung auf den Blättern ermittelt.

Das Ergebnis des Versuches zeigt, dass beispielsweise der Wirkstoff 4 als 0,025%ige Wirkstoffbrühe eine bessere fungizide Wirkung hat (beispielsweise 97%ige Wirkung) als der bekannte Wirkstoff A (beispielsweise 60%ige Wirkung).

*Versuch 3*

*Wirksamkeit gegen Gurkenmehltau*

Blätter von in Töpfen gewachsenen Gurkenkeimlingen der Sorte Chinesische Schlange werden im Zweiblattstadium mit einer Sporensuspension des Gurkenmehltaus *(Erysiphe cichoracearum)* besprüht. Nach etwa 20 h werden die Versuchspflanzen mit wässeriger Spritzbrühe, die 80% Wirkstoff und 20% Emulgiermittel in der Trockensubstanz enthält, bis zur Tropfnässe besprüht. Nach dem Antrocknen des Spritzbelages werden sie anschliessend im Gewächshaus bei Temperaturen zwischen 20 und 22° C und 70 bis 80% relativer Luftfeuchtigkeit aufgestellt. Zur Beurteilung der Wirksamkeit der neuen Stoffe wird das Ausmass der Pilzentwicklung nach 21 d beurteilt.

Das Ergebnis des Versuches zeigt, dass beispielsweise die Wirkstoffe 3 und 4 als 0,025%ige Wirkstoffbrühen eine gute fungizide Wirkung haben (beispielsweise 100%ige Wirkung).

**Patentansprüche** für die Vertragsstaaten BE, CH, DE, FR, GB, IT, LI, NL, SE

1. Phenylketenacetal der Formel (1):

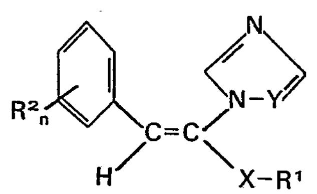

(1)

in der

$R^1$ Alkyl mit 1 bis 6 C-Atomen, Cycloalkyl mit 3 bis 8 C-Atomen, Naphthyl oder Phenyl bedeutet, wobei der Phenylrest einfach oder mehrfach durch Alkyl oder Alkoxy mit jeweils 1 bis 5 C-Atomen, Fluor, Chlor, Brom, Jod oder Trifluormethyl oder durch Phenoxy substituiert sein kann,

$R^2$ Wasserstoff, Alkyl oder Alkoxy mit jeweils 1 bis 5 C-Atomen, Fluor, Chlor, Brom, Jod oder Trifluormethyl bedeutet,

n 1 oder 2 bedeutet,

Y für N und X für O oder S steht,

sowie deren Salze und Metallkomplexe.

2. Fungizid enthaltend ein Phenylketenacetal gemäss Anspruch 1.

3. Fungizid enthaltend einen festen oder flüssigen Trägerstoff und ein Phenylketenacetal gemäss Anspruch 1.

4. Verfahren zur Herstellung eines Fungizides, dadurch gekennzeichnet, dass man einen festen oder flüssigen Trägerstoff vermischt mit einem Phenylketenacetal gemäss Anspruch 1.

5. Verfahren zur Bekämpfung von Pilzen, dadurch gekennzeichnet, dass man die Pilze oder die vor Pilzbefall zu schützenden Gegenstände behandelt mit einem Phenylketenacetal gemäss Anspruch1.

6. Verfahren zur Herstellung eines Phenylketenacetals gemäss Anspruch 1, dadurch gekennzeichnet, dass man ein Sulfonat der allgemeinen Formel (2):

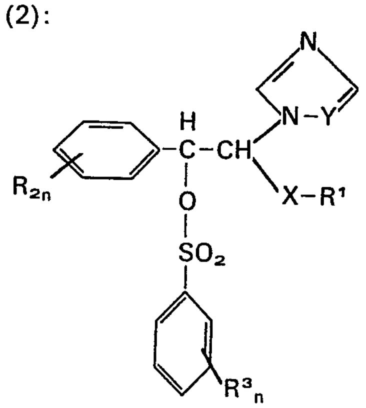

(2)

worin $R^1$, $R^2$, X, Y und n die in Anspruch 1 angegebene Bedeutung haben und $R^3$ Wasserstoff, Halogen oder Alkyl mit 1 bis 5 C-Atomen bedeutet, in Gegenwart eines Lösungsmittels und eines basischen Katalysators bei Temperaturen zwischen 10 und 100° C zersetzt.

7. Phenylketenacetal gemäss Anspruch 1, dadurch gekennzeichnet, dass $R^1$ Wasserstoff, X Sauerstoff, Y Stickstoff und n 1 bedeutet.

8. Fungizid enthaltend ein Phenylketenacetal definiert wie in Anspruch 1, dadurch gekennzeichnet, dass $R^1$ Monochlorphenyl oder Dichlorphenyl, $R^2$ Wasserstoff, X Sauerstoff, Y Stickstoff und n 1 bedeutet.

**Patentansprüche** für den Vertragsstaat: AT

1. Fungizid enthaltend ein Phenylketenacetal der Formel (1):

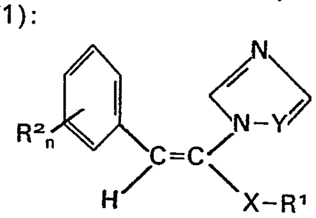

(1)

in der

$R^1$ Alkyl mit 1 bis 6 C-Atomen, Cycloalkyl mit 3 bis 8 C-Atomen, Naphthyl oder Phenyl bedeutet, wobei der Phenylrest einfach oder mehrfach durch Alkyl oder Alkoxy mit jeweils 1 bis 5 C-Atomen, Fluor, Chlor, Brom, Jod, Trifluormethyl oder durch Phenoxy substituiert sein kann,

$R^2$ Wasserstoff, Alkyl oder Alkoxy mit jeweils 1 bis 5 C-Atomen, Fluor, Chlor, Brom, Jod oder Trifluormethyl bedeutet,

n 1 oder 2 bedeutet,

Y für N und X für O oder S steht,

sowie deren Salze und Metallkomplexe.

2. Fungizid enthaltend einen festen oder flüssigen Trägerstoff und ein Phenylketenacetal gemäss Anspruch 1.

3. Verfahren zur Herstellung eines Fungizides, dadurch gekennzeichnet, dass man einen festen oder flüssigen Trägerstoff vermischt mit einem Phenylketenacetal gemäss Anspruch 1.

4. Verfahren zur Bekämpfung von Pilzen, dadurch gekennzeichnet, dass man die Pilze oder die vor Pilzbefall zu schützenden Gegenstände behandelt mit einem Phenylketenacetal gemäss Anspruch 1.

5. Verfahren zur Herstellung eines Phenylketenacetals gemäss Anspruch 1, dadurch gekennzeichnet, dass man ein Sulfonat der allgemeinen Formel (2):

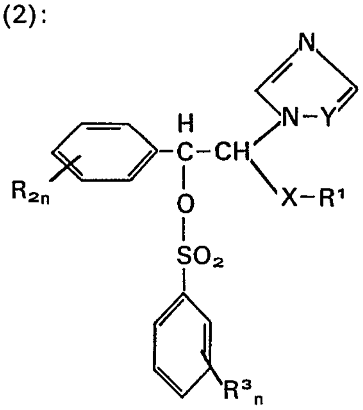

(2)

worin $R^1$, $R^2$, X, Y und n die in Anspruch 1 angegebene Bedeutung haben und $R^3$ Wasserstoff, Halogen oder Alkyl mit 1 bis 5 C-Atomen bedeutet, in Gegenwart eines Lösungsmittels und eines basischen Katalysators bei Temperaturen zwischen 10 und 100°C zersetzt.

6. Fungizid enthaltend ein Phenylketenacetal definiert wie in Anspruch 1, dadurch gekennzeichnet, dass $R^1$ Monochlorphenyl oder Dichlorphenyl, $R^2$ Wasserstoff, X Sauerstoff, Y Stickstoff und n 1 bedeutet.

**Claims** for the contracting States BE, CH, DE, FR, GB, IT, LI, NL, SE

1. A phenylketene acetal of the formula (1):

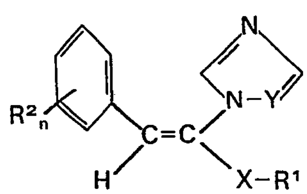

(1)

where:

$R^1$ is alkyl of 1 to 6 carbon atoms, cycloalkyl of 3 to 8 carbon atoms, naphthyl or phenyl, the phenyl radical being unsubstituted or mono- or polysubstituted by alkyl or alkoxy, each having 1 to 5 carbon atoms, fluorine, chlorine, bromine, iodine, trifluoromethyl or phenoxy;

$R^2$ is hydrogen, alkyl or alkoxy, each having 1 to 5 carbon atoms, fluorine, chlorine, bromine or trifluoromethyl;

n is 1 or 2;

Y is N; and

X is O or S,

and its salts and metal complexes.

2. A fungicide containing a phenylketene acetal as claimed in Claim 1.

3. A fungicide containing a solid or liquid carrier and a phenylketene acetal as claimed in Claim 1.

4. A process for producing a fungicide, wherein a solid or liquid carrier is mixed with a phenylketene acetal as claimed in Claim 1.

5. A process for combating fungi, wherein the fungi or the objects to be protected against fungal attack are treated with a phenylketene acetal as claimed in Claim 1.

6. A process for preparing a phenylketene acetal as claimed in Claim 1, wherein a sulphonate of the general formula (2):

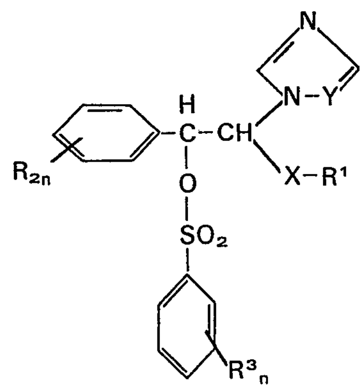

(2)

where $R^1$, $R^2$, X, Y and n have the meanings given in Claim 1, and $R^3$ is hydrogen, halogen or alkyl of 1 to 5 carbon atoms, is decomposed in the presence of a solvent and a basic catalyst at a temperature of from 10 to 100°C.

7. A phenylketene acetal as claimed in Claim 1, where $R^1$ is hydrogen, X is oxygen, Y is nitrogen and n is 1.

8. A fungicide containing a phenylketene acetal as defined in Claim 1, wherein $R^1$ is monochlorophenyl or dichlorophenyl, $R^2$ is hydrogen, X is oxygen, Y is nitrogen and n is 1.

**Claims** for the contracting State: AT

1. A fungicide containing a phenylketene acetal of the formula (1):

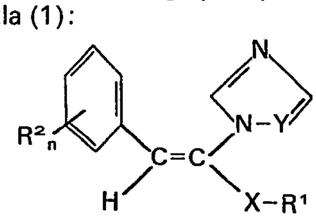

(1)

where:

$R^1$ is alkyl of 1 to 6 carbon atoms, cycloalkyl of 3 to 8 carbon atoms, naphthyl or phenyl, the phenyl radical being unsubstituted or mono- or polysubstituted by alkyl or alkoxy, each having 1 to 5 carbon atoms, fluorine, chlorine, bromine, iodine, trifluoromethyl or phenoxy;

$R^2$ is hydrogen, alkyl or alkoxy, each having 1 to 5 carbon atoms, fluorine, chlorine, bromine or trifluoromethyl;

n is 1 or 2;

Y is N; and

X is O or S,

or a salt or metal complex thereof.

2. A fungicide containing a solid or liquid carrier and a phenylketene acetal as defined in Claim 1.

3. A process for producing a fungicide, wherein a solid or liquid carrier is mixed with a phenylketene acetal as defined in Claim 1.

4. A process for combating fungi, wherein the fungi or the objects to be protected against fungal attack are treated with a phenylketene acetal as defined in Claim 1.

5. A process for preparing a phenylketene acetal as defined in Claim 1, wherein a sulphonate of the general formula (2):

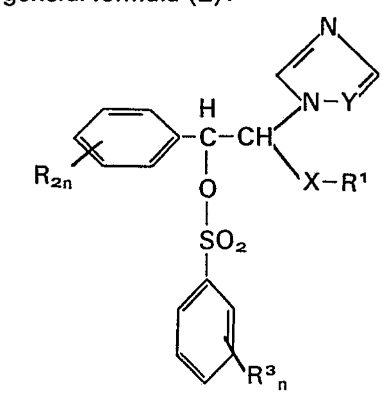

(2)

where $R^1$, $R^2$, X, Y and n have the meanings given in Claim 1, and $R^3$ is hydrogen, halogen or alkyl of 1 to 5 carbon atoms, is decomposed in the presence of a solvent and a basic catalyst at a temperaure of from 10 to 100°C.

6. A fungicide containing a phenylketene acetal as defined in Claim 1, where $R^1$ is monochlorophenyl or dichlorophenyl, $R^2$ is hydrogen, X is oxygen, Y is nitrogen and n is 1.

**Revendications** pour les Etats contractants BE, CH, DE, FR, GB, IT, LI, NL, SE

1. Phénylcéténacétal de formule (1):

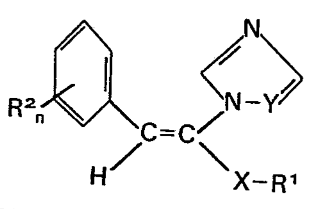

(1)

dans laquelle:

$R^1$ représente alkyle de 1 à 6 atomes C, cycloalkyle de 3 à 8 atomes C, naphtyle ou phényle, le reste phényle pouvant être substitué une fois ou plusieurs fois par alkyle ou alcoxy de chacun 1 à 5 atomes C, fluor, chlore, brome, iode ou trifluorométhyle, ou par phénoxy;

$R^1$ représente hydrogène, alkyle ou alcoxy, de chacun 1 à 5 atomes C, fluor, chlore, brome, iode ou trifluorométhyle;

n représente 1 ou 2;

Y est mis pour N, et

X pour O ou S,

ainsi que ses sels et complexes métalliques.

2. Fongicide contenant un phénylcéténacétal selon la revendication 1.

3. Fongicide contenant un support solide ou liquide et un phénylcéténacétal selon la revendication 1.

4. Procédé de préparation d'un fongicide, caractérisé par le fait que l'on mélange un support solide ou liquide avec un phénylcéténacétal selon la revendication 1.

5. Procédé de lutte contre les champignons, caractérisé par le fait que l'on traite les champignons, ou les objets à protéger des champignons, avec un phénylcéténacétal selon la revendication 1.

6. Procédé de préparation d'un phénylcéténacétal selon la revendication 1, caractérisé par le fait que l'on décompose un sulfonate de formule générale (2):

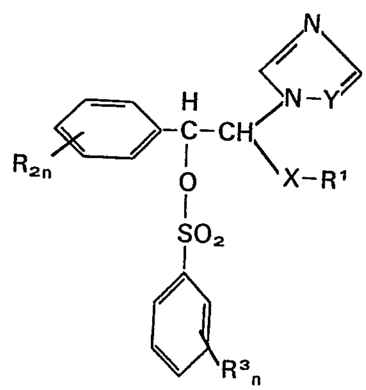

(2)

où $R^1$, $R^2$, X, Y et n ont les significations indiquées dans la revendication 1 et $R^3$ représente hydrogène, halogène ou alkyle de 1 à 5 atomes C, en présence d'un solvant et d'un catalyseur basique, à des températures comprises entre 10 et 100°C.

7. Phénylcéténacétal selon la revendication 1, caractérisé par le fait que $R^1$ représente hydrogène, X oxygène, Y azote et n 1.

8. Fongicide contenant un phénylcéténacétal défini comme dans la revendication 1, caractérisé par le fait que $R^1$ représente monochlorophényle ou dichlorophényle, $R^2$ hydrogène, X oxygène, Y azote et n 1.

**Revendications** pour l'Etat contractant: AT

1. Fongicide contenant un phénylcéténacétal de formule (1):

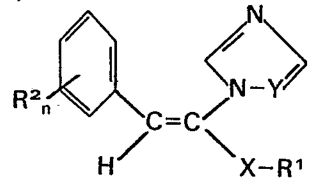

(1)

dans laquelle:

$R^1$ représente alkyle de 1 à 6 atomes C, cycloalkyle de 3 à 8 atomes C, naphtyle ou

phényle, le reste phényle pouvant être substitué une fois ou plusieurs fois par alkyle ou alcoxy de chacun 1 à 5 atomes C, fluor, chlore, brome, iode ou trifluorométhyle, ou par phénoxy;

$R^2$ représente hydrogène, alkyle ou alcoxy, de chacun 1 à 5 atomes C, fluor, chlore, brome, iode ou trifluorométhyle;

n représente 1 ou 2;

Y est mis pour N, et

X pour O ou S,

ainsi que ses sels et complexes métalliques.

2. Fongicide contenant un support solide ou liquide et un phénylcéténacétal selon la revendication 1.

3. Procédé de préparation d'un fongicide, caractérisé par le fait que l'on mélange un support solide ou liquide avec un phénylcéténacétal selon la revendication 1.

4. Procédé de lutte contre les champignons, caractérisé par le fait que l'on traite les champignons, ou les objets à protéger des champignons, avec un phénylcéténacétal selon la revendication 1.

5. Procédé de préparation d'un phénylcéténacétal selon la revendication 1, caractérisé par le fait que l'on décompose un sulfonate de formule générale (2):

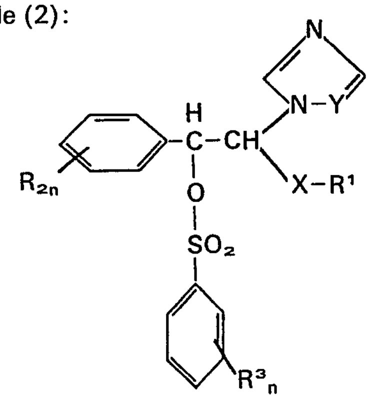

(2)

où $R^1$, $R^2$, X, Y et n ont les significations indiquées dans la revendication 1 et $R^3$ représente hydrogène, halogène ou alkyle de 1 à 5 atomes C, en présence d'un solvant et d'un catalyseur basique, à des températures comprises entre 10 et 100°C.

6. Fongicide contenant un phénylcéténacétal défini comme dans la revendication 1, caractérisé par le fait que $R^1$ représente monochlorophényle ou dichlorophényle, $R^2$ hydrogène, X oxygène, Y azote et n 1.